# DEMANDE DE BREVET EUROPEEN

(11) **EP 0 653 193 A1**
(43) Date de publication de la demande: **17.05.1995**
(21) Numéro de dépôt: 94402537.8
(22) Date de dépôt: 09.11.1994
(51) Int. Cl.: A61F 2/02, A61F 2/06

(54) **Dispositif de repérage sous-cutané d'une prothèse médicale et prothèse correspondante**

(30) Priorité: 16.11.1993 FR 9313668
(71) Demandeur: B. BRAUN CELSA, Société Anonyme, F-86360 Chasseneuil du Poitou (FR)
(72) Inventeur: Roussigne, Maurice, F-86000 Poitiers (FR); Nadal, Guy, F-86000 Poitiers (FR); Bovyn, Gilles, F-22000 Saint-Brieuc (FR)
(74) Mandataire: Lerner, François

(57) **Abrégé**

Il s'agit d'un dispositif de repérage sous-cutané d'une prothèse pourvue d'un élément tubulaire, ou cathéter, (3) perforable adapté pour être implanté dans le corps d'un patient.

Selon l'invention, le dispositif comprend une enveloppe extérieure (15) présentant un passage principal (25) la traversant suivant un axe, pour recevoir à travers lui l'élément tubulaire et un clou (17) radial monté glissant dans l'enveloppe pour perforer, sous la commande d'un opérateur agissant sur lui, l'élément tubulaire et bloquer l'enveloppe de long de celui-ci.

Application notamment au repérage des filtres sanguins temporaires.

## Description

L'invention se rapporte à un dispositif de repérage sous-cutané d'une prothèse médicale comprenant un élément tubulaire perforable et implantable dans le corps d'un patient, le dispositif de l'invention étant tout particulièrement applicable au repérage par palpation, à travers la peau du patient, d'un cathéter ou équivalent habituellement associé à un filtre sanguin temporaire.

Spécialement dans le domaine des prothèses vasculaires qui doivent pouvoir être retirées du corps au bout d'un certain temps, lorsque leur présence dans le vaisseau n'est plus utile, les cathéters, (ou tiges flexibles) auxquels elles sont généralement fixées pour permettre en particulier leur retrait, présentent dans certain cas des caractéristiques particulières.

Ainsi, en particulier dans le domaine des filtres sanguins temporaires, il est offert aujourd'hui la possibilité au praticien, par exemple lorsqu'une implantation du filtre doit se prolonger pendant plusieurs semaines, de couper à longueur le cathéther, après avoir implanté le filtre, de telle sorte que l'extrémité proximale de ce cathéter (extrémité opposée à celle portant le filtre) ne débouche pas à l'extérieur du corps du patient. Après avoir coupé cette extrémité, le praticien fixe autour du cathéter un organe de repérage qu'il va disposer avec ladite extrémité du cathéter dans un petit logement qu'il a ménagé sous la peau du patient, à proximité immédiate de la voie d'accès qui a été utilisée pour implanter le filtre. Après quoi il ne lui reste qu'à suturer les chairs, en laissant enfoui le filtre et son cathéter, avec son organe de repérage. Lorsque le filtre devra être retiré, il suffira alors au praticien de détecter, par exemple par palpations, l'emplacement de l'organe de repérage auquel il pourra accéder par une nouvelle petite incision, ceci lui permettant d'accéder au cathéter qu'il pourra ainsi retirer, voire déplacer.

Dans la technique, on connaît déjà de tels dispositifs de repérage sous-cutané. La demande de brevet FR 9000769 (ou le brevet américain correspondant 07/731536 du 17 Juillet 1991 ) en offre un exemple. Un autre exemple peut être trouvé au brevet US 4834713.

On notera également qu'outre les filtres sanguins temporaires, l'art antérieur offre d'autres types de prothèses médicales associées à des cathéters ou équivalents et donc susceptibles de se voir adjoindre un dispositif de repérage sous-cutané (voir publication EP 92402098.5).

Tels qu'ils sont présentés, les deux dispositifs de repérage susmentionnés paraissent toutefois présenter certaines difficultés d'utilisation.

Ainsi, les différentes versions du "bouton" décrit au brevet US 4834713 prévoient, pour sa fixation au cathéter porteur, l'écrasement d'une bague intérieure métallique, voire l'utilisation d'une réduction de section locale du passage interne du bouton à travers lequel passe le cathéter.

Dans la mesure où certains efforts de traction peuvent être exercés sur les cathéters porteurs une fois implantés, la retenue des dispositifs de repérage par simple restriction de section peut s'avérer dangereuse pour le patient. De la même manière, l'écrasement local du dispositif pour permettre sa fixation autour du cathéter peut s'avérer délicate, le praticien pouvant avoir quelques difficultés à apprécier la force d'écrasement à appliquer, qui doit être ni trop faible (pour assurer le maintien), ni trop forte (pour éviter d'endommager la pièce de repérage ou le cathéter).

Des problèmes comparables peuvent se poser dans le cadre des solutions envisagées à la publication FR 9000769. En particulier, la dernière solution privilégiée au brevet US 07/731536 (consistant en l'utilisation d'un bouchon pouvant être éventuellement vissé dans le passage interne du cathéter, du côté de son extrémité proximale), peut en pratique s'avérer délicate, le praticien pouvant avoir des difficultés à juger du bon engagement du bouchon.

Compte tenu notamment de ces solutions existantes, l'invention a tout particulièrement pour objet :
- de proposer un dispositif de repérage sous-cutané d'un élément tubulaire de prothèse implantable qui soit aisément toléré par le patient (non agressif),
- d'assurer une sécurité et une fiabilité de positionnement du dispositif de repérage autour du cathéter ou équivalent,
- de faciliter l'utilisation de ce dispositif par le praticien, de telle sorte que celui-ci puisse aisément être sûr de son verrouillage, sans risque particulier, ni pour le patient, ni pour la prothèse,
- d'autoriser éventuellement un désengagement du dispositif de repérage, devant permettre soit son retrait de l'élément tubulaire, soit son changement de position le long de celui-ci, et ceci dans le respect des exigences précédentes,
- de permettre une réalisation industriellement satisfaisante du dispositif, et même de la prothèse dans son ensemble.
   Dans le but de satisfaire au moins pour l'essentiel les points énumérés ci-dessus, l'invention propose donc un dispositif de repérage sous-cutané perfectionné tel qu'il se caractérise essentiellement en ce qu'il comprend :
- une enveloppe en matière biocompatible qui présente un passage principal traversant ladite enveloppe suivant un axe, pour recevoir à travers lui ledit élément tubulaire,
- et un clou de blocage disposé suivant une direction transversale à l'axe du passage principal de l'enveloppe dans laquelle ledit clou est monté glissant suivant cette dite direction transversale, pour perforer l'élément tubulaire sous la commande d'un opérateur agissant sur lui, en bloquant alors axialement le dispositif de repérage vis-à-vis de l'élément tubulaire.

Pour favoriser un bon guidage du clou vis-à-vis de l'enveloppe, une caractéristique complémentaire de l'invention prévoit qu'avantageusement cette enveloppe renfermera une bague interne non prévue pour être déformée et présentant une paroi entourant un passage interne coaxial avec celui de ladite enveloppe, cette bague étant disposée de telle sorte que ledit clou de blocage traverse sa paroi pour venir perforer l'élément tubulaire.

Avantageusement, tant le clou que la bague seront totalement enrobés par l'enveloppe. De cette manière, ils pourront en particulier être réalisés en métal. Notamment dans ce cas, on pourra en outre préférer réaliser ladite enveloppe en un matériau déformable, relativement souple, de telle sorte que l'opérateur puisse agir depuis l'extérieur de l'enveloppe, par exemple manuellement, sur le déplacement du clou, la matière utilisée pouvant au demeurant être choisie pour que l'opérateur puisse, à travers elle, distinguer en particulier le clou, afin de pouvoir plus facilement juger de sa position engagée ou non dans l'élément tubulaire.

Pour la manoeuvre de ce clou, une autre caractéristique de l'invention envisage qu'on peut lui associer un piston de manoeuvre, plus volumineux que lui, donc plus aisément manoeuvrable et repérable à travers l'enveloppe, un passage intérieur devant alors bien entendu être prévu dans l'enveloppe pour son déplacement suivant la direction transversale d'action du clou.

Selon une caractéristique complémentaire, ledit clou pourra par ailleurs présenter une section nettement inférieure au diamètre interne du cathéter, de manière en particulier à ne pas fragiliser le cathéter-support.

Avant de décrire un mode préféré de réalisation du dispositif de l'invention, on notera encore que pour la sécurité du patient, le dispositif sera de préférence dépourvu de moyens d'ancrage au corps de ce patient, évitant notamment que des chairs puissent être tirées voire arrachées.

Par souci de clarté, la description qui va suivre va être faite en relation avec les dessins annexés, donnés uniquement à titre d'exemples non limitatifs, et dans lesquels :
- la figure 1 montre suivant une vue schématique générale, une disposition possible du dispositif de repérage sous-cutané de l'invention du côté de l'extrémité proximale d'une prothèse vasculaire,
- la figure 2 est une vue de détail en coupe transversale médiane de la partie repérée II sur la figure 1 (coupe faite suivant la ligne II-II de la figure 3),
- la figure 3 est une vue de dessus (complétée par symétrie) dans le sens de la flèche III de la figure 2,
- et, tandis que la figure 2 montre le dispositif de repérage avec son clou non engagé, la figure 4 montre, suivant la même vue en coupe, le même dispositif avec son clou venant perforer la paroi du cathéter.

Le dispositif de repérage de l'invention pouvant tout particulièrement être associé à un instrument de filtration sanguine temporaire, on ne décrira ci-après que ce mode de réalisation, les indications contenues dans la présente demande ainsi que les connaissances techniques de l'homme du métier devant lui permettre d'adapter aisément le dispositif pour une utilisation, par exemple, sur un dispositif d'atérectomie, du type de celui de la publication EP 92 402098.5.

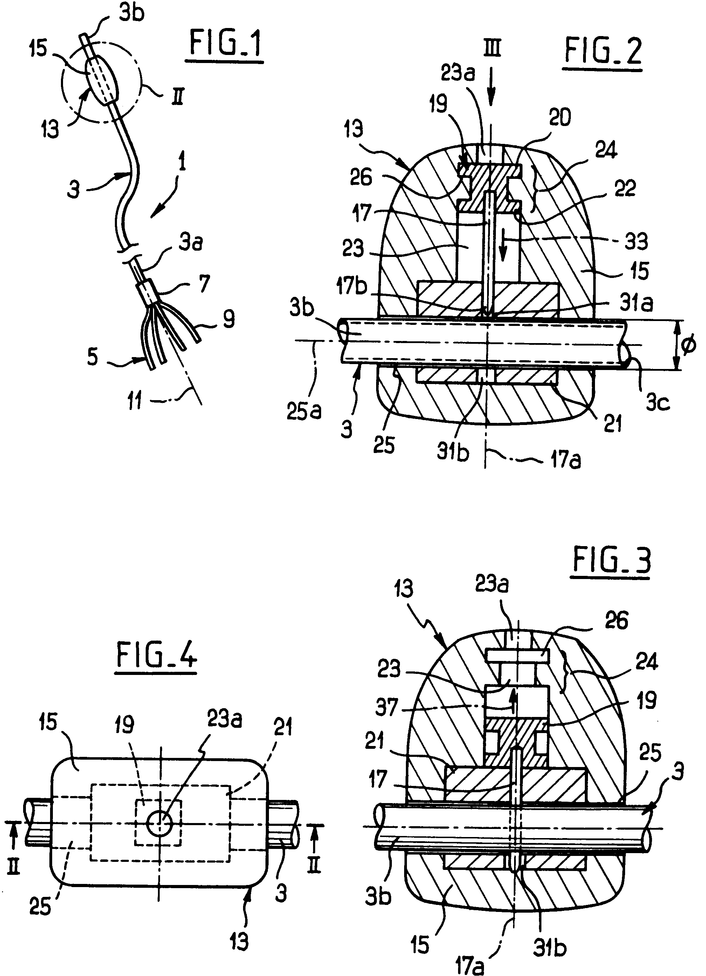

Sur la figure 1, on voit donc illustrée schématiquement une unité de filtration temporaire 1, tout à fait classique en soi, comprenant un cathéter 3 (ou tout moyen souple, flexible équivalent, tel qu'une tige ou un filament) portant, fixé à lui du côté de son extrémité distale 3a, un filtre sanguin 5, lequel est en l'espèce constitué d'une tête de liaison 7, par exemple sertie à l'extrémité 3a et dans laquelle sont réunies une série de pattes allongées 9 s'étendant essentiellement suivant la direction générale 11 du dispositif. Pour assurer leur rôle de filtration sanguine, ces pattes sont expansibles radialement à l'axe 11, de telle sorte qu'à partir de leur extrémité libre opposée à la tête 7, elles se déploient suivant une corolle sensiblement conique.

Pour tout renseignement complémentaire relatif au filtre ou au cathéter, on pourra se reporter à la publication américaine US 07/731536 ou encore FR 92 09957 qui sont introduites dans la présente demande par référence.

Concernant le cathéter 3, on notera encore toutefois qu'il est réalisé en une matière biocompatible, telle par exemple qu'une silicone, pour s'adapter en souplesse aux méandres du chemin d'implantation, ou voie d'accès, qu'il doit suivre pour parvenir à l'endroit d'installation prévu dans le vaisseau.

Conformément à l'invention, ce cathéter doit en outre être perforable. Du côté de son extrémité proximale 3b, il est par ailleurs équipé d'un dispositif de repérage 13 pouvant extérieurement présenter une forme arrondie, par exemple en olive dépourvue d'angles vifs.

Comme illustré en particulier sur la figure 2, le dispositif 13 comprend ici une enveloppe extérieure 15 en matériau biocompatible avantageusement souple, ou déformable. A ce sujet, on pourra notamment prévoir de réaliser l'enveloppe en silicone ou équivalent pouvant présenter une dureté comprise entre environ 35 à 40 et 90 à 95 shores A et de préférence, de l'ordre de 50 à 70 shores A.

Dans la version illustrée, cette enveloppe ou gaine extérieure 15 renferme un clou 17 dont le rôle va être de bloquer en position le dispositif 13 le long du cathéter 3, ainsi qu'un piston 19 de manoeuvre dudit clou et une bague 21 à travers laquelle le clou va passer sensiblement radialement pour perforer le cathéter. Il doit être clair que l'on aurait toutefois pu réduire la section du passage 23 réservé ici au guidage en translation du piston 19, de sorte à adapter la section de ce passage au diamètre du clou 17, dans le but de se passer du piston tout en favorisant le guidage du clou suivant son axe de déplacement 17a. On aurait également pu prévoir de faire ressortir, dans sa position non engagée de la figure 2, le clou 17 hors de l'enveloppe 15, de manière à permettre alors au praticien-opérateur de pouvoir directement venir pousser sur le clou pour l'engager à travers la paroi du cathéter jusqu'à sa position de la figure 4.

Quelle que soit la variante choisie, la direction de déplacement du clou est transversale, et de préférence sensiblement perpendiculaire, à l'axe 25a du passage intérieur principal 25 de l'enveloppe dont le diamètre ⌀ sera de préférence très légèrement supérieur au diamètre du cathéter afin de permettre à l'origine d'enfiler aisément l'olive 13 autour de son extrémité proximale 3b.

Dans la réalisation illustrée, le clou 17 s'étend exclusivement à l'intérieur du passage secondaire transversal 23, d'axe 17a, de l'enveloppe, en étant lié (par exemple engagé à force et/ou collé), du côté opposé à sa pointe de perforation 17b, au piston 19 qui est donc adapté pour glisser également dans le passage 23. A ce sujet, on remarquera que le piston pourra présenter, pour faciliter sa manoeuvre, une forme de cylindre en "I" suivant une coupe faite dans un plan contenant l'axe 17a (plan de coupe de la figure 2).

Notamment pour éviter toute manoeuvre malencontreuse du piston et/ou du clou, le passage 23 qui peut communiquer exclusivement, en 23a, avec l'extérieur pourra présenter localement une zone 24 de retenue du piston en position non engagée du clou (position de la figure 2).

Si la bague 21 n'est pas réalisée en un matériau perforable, elle présentera, dans le prolongement du passage 23, une ouverture radiale traversant la bague de préférence de part en part, le diamètre de cette ouverture pouvant n'être que très légèrement supérieur à celui du clou pour la partie 31a dans laquelle le clou est en l'espèce en permanence engagé, tandis que du côté diamétralement opposé 31b, le diamètre de l'ouverture peut être plus large.

Le positionnement optimal de l'enveloppe 15 peut s'effectuer comme suit : après avoir enfilé correctement l'enveloppe autour du cathéter, le praticien vient appuyer avec ses doigts sur la partie de celle-ci qui entoure le piston 19, lequel est alors dans sa position "arrière" de la figure 2 (clou à proximité immédiate de la paroi 3c du cathéter). Le praticien doit appuyer suffisamment pour le dégager le piston de sa zone de retenue 24 dont notamment l'épaulement 26 retient la semelle arrière 20 du piston. Dans la mesure où l'on aura de préférence choisi la matière de l'enveloppe pour que celle-ci soit déformable, l'effort exercé sur elle va avoir tendance à déformer la zone d'entourage du piston, jusqu'à le libérer pour que, sous cette poussée, il coulisse dans le sens de la flèche 33 de la figure 2, ses semelles 20, 22 assurant son guidage. Entraîné par le piston, le clou perfore alors la paroi 3c du cathéter, jusqu'à ressortir du côté diamétralement opposé dans l'orifice 31b. Le piston est alors en position "avant" de la figure 4, en butée contre la bague 21.

On notera que si la matière de l'enveloppe a été choisie translucide, ou transparente, le praticien pourra s'assurer du bon engagement du clou à la fois par la sensation tactile ressentie et par une vérification visuelle. Quoi qu'il en soit, la perforation du cathéter par le clou aura fixé longitudinalement la position de l'enveloppe.

Pour éviter d'affaiblir le cathéter 3 (sans donc l'obturer), il pourrait être avantageux de prévoir un diamètre de clou 17 inférieur au diamètre intérieur du cathéter. Un rapport de 3 à 4 peut être approprié.

Si la matière de l'enveloppe 15 est réellement souple, le praticien pourra éventuellement, par une nouvelle pression extérieure sur cette enveloppe, entraîner le piston à contresens (flèche 37 de la figure 4) jusqu'à ce qu'il retourne avec le clou à sa suite dans sa position arrière de la figure 2, permettant ainsi de repositionner si nécessaire le dispositif 13 en un autre endroit le long du cathéter. Il doit toutefois être clair que rien n'oblige à ce que cette possibilité de retour en arrière du piston soit permise.

## Revendications

1. Dispositif de repérage sous-cutané d'une prothèse médicale, laquelle comporte un élément tubulaire (3) perforable, adapté pour être implanté dans le corps d'un patient, ledit dispositif comprenant :
- une enveloppe extérieure (15) présentant un passage principal (25) la traversant suivant un axe, pour recevoir à travers lui l'élément tubulaire,
- et un clou (17) disposé suivant une direction transversale à l'axe (25a) du passage principal (25) de l'enveloppe dans laquelle ledit clou est monté glissant suivant cette dite direction transversale, pour perforer l'élément tubulaire sous la commande d'un opérateur agissant sur lui et bloquer l'enveloppe le long de cet élément.

2. Dispositif de repérage sous-cutané d'une prothèse (1) propre à être implantée de manière amovible dans le corps d'un patient pour y filtrer le sang, ladite prothèse comprenant un cathéter (3)présentant une extrémité distale où est fixée un filtre sanguin (5) et une extrémité proximale vers laquelle est fixé ledit dispositif de repérage, lequel comprend :
- une enveloppe (15) présentant un passage principal (25) la traversant suivant un axe pour recevoir à travers lui ledit cathéter,
- et des moyens de blocage de ladite enveloppe le long de ce cathéter,
caractérisé en ce que lesdits moyens de blocage comprennent un clou (17) disposé suivant une direction transversale (17a) à l'axe (25a) du passage principal (25) de l'enveloppe dans laquelle ledit clou est monté glissant suivant cette dite direction transversale pour perforer le cathéter.

3. Dispositif selon la revendication 1 ou la revendication 2, caractérisé en ce que la section du clou (17) est inférieure à la section intérieure de l'élément tubulaire, ou cathéter, (3).

4. Dispositif selon l'une quelconque des revendications précédentes, caractérisé en ce que l'enveloppe (15) renferme une bague (21) propre à recevoir à travers elle ledit élément tubulaire, ou cathéter, la bague présentant un passage transversal (31a, 31b) à travers lequel est monté glissant le clou (17).

5. Dispositif selon l'une quelconque des revendications précédentes, caractérisé en ce que l'enveloppe (15) est réalisée en une matière souple déformable, telle qu'une silicone, à travers laquelle un opérateur peut agir sur le déplacement du clou (17), lequel est entièrement contenu à l'intérieur de l'enveloppe.

6. Dispositif selon l'une des revendications 1 à 5, caractérisé en ce que la dureté de l'enveloppe (15) est de l'ordre de 50 à 70 Shores A.

7. Dispositif selon la revendication 5 ou la revendication 6, caractérisé en ce que l'enveloppe (15) renferme en outre un piston de manoeuvre (19) lié au clou (17), ledit piston étant déplaçable sous l'effet d'une pression exercée depuis l'extérieur, à travers l'enveloppe (15), dans un espace intérieur (23) de cette dernière dirigé suivant la direction transversale (17a) de déplacement du clou.

8. Dispositif selon la revendication 7, caractérisé en ce que le piston de manoeuvre (19) se présente sous la forme générale d'un cylindre d'axe parallèle à celui du clou (17) ayant une forme de "I" en section faite selon un plan contenant ledit axe (17a) du clou.

9. Dispositif selon l'une quelconque des revendications précédentes, caractérisé en ce qu'il est dépourvu de moyens d'ancrage au corps du patient.

10. Prothèse médicale adaptée pour être implantée dans le corps d'un patient et comportant un élément tubulaire, ou cathéter, perforable (3), caractérisée en ce qu'elle est équipée d'un dispositif de repérage sous-cutané (13) selon l'une quelconque des revendications précédentes, propre à la localisation dudit élément tubulaire à travers la peau du patient, après que la prothèse a été implantée.
